# EUROPEAN PATENT APPLICATION

(11) **EP 0 621 010 A2**
(43) Date of publication of application: **26.10.1994**
(21) Application number: 94250062.0
(22) Date of filing: 09.03.1994
(51) Int. Cl.: A61B 17/60

(54) **Surgical cable leader and terminations**

(30) Priority: 21.04.1993 US 52191
(71) Applicant: AMEI TECHNOLOGIES Inc., Wilmington, Delaware 19899 (US)
(72) Inventor: Foley, Kevin T., Memphis, Tennessee 38119 (US); Preissman, Howard E., Dallas, Texas 75240 (US)
(74) Representative: UEXKÜLL & STOLBERG

(57) **Abstract**

A surgical cable assembly (10) with a leader (11) is disclosed for guiding surgical cables (14, 16) in medical procedures. Leader (11) includes a stem (18) with two or more arms (20, 22) extending from stem (18). Surgical cables (14, 16) are attached to arms (20, 22) so that when stem (18) is passed through a selected region surgical cables (14, 16) attached to the arms will be passed through the selected region as well. End terminations (28, 30, 32) for surgical cables (12, 14, 16) are also disclosed.

## Description

### TECHNICAL FIELD OF THE INVENTION

This invention relates to surgically implanted wires and cables and more particularly relates to improved methods and apparatus for use in surgically installing wires and cables at selected locations in a patient's body.

### BACKGROUND OF THE INVENTION

Surgical wires and cables are used in a variety of surgical procedures, for example, reconstructive spine surgery such as fusions, spine trauma surgery, total hip arthroplasty, fracture fixation, open heart surgery for closures of the sternum, oral and facial surgery to fix mandibular fractures and the like, and other surgical procedures. Often, surgical cables and wires are used to encircle or loop about bones to hold them together for healing or fusion in some types of spinal surgery. For purposes of this application, "cable" includes monofilament and single strand wire along with multifilament and multistrand surgical cable and wire ropes.

For some surgical procedures it is desirable to pass two surgical cables under the lamina (sublamina) of one or more vertebrae. Once the surgical cables are passed under the lamina of a vertebra, the surgical cable is frequently pulled through and then secured in a loop about the vertebra. This latter procedure may be done, for example, to allow the vertebra to fuse. In performing this type of procedure, it is preferable to pass surgical cables or objects through the sublamina region a minimum number of times.

One method of passing two surgical cables while only guiding one item through the sublamina region has been to connect two surgical cables with a monofilament member. Prior to inserting in the sublamina region, the monofilament member is bent to form a V-configuration with the surgical cables extending from the ends of the monofilament member. The monofilament member is then passed through the sublamina region to position the desired portion of the surgical cables relative to the lamina. The monofilament member is then cut resulting in two separate surgical cables under the lamina.

This previously known method has some disadvantages. First, bending the single monofilament member to form a V-configuration means that the portion of the monofilament member that leads the surgical cables under the lamina is twice the width of the monofilament member. The double width of the bent monofilament member may make it difficult to guide at times. Additionally, bending the monofilament member so that both surgical cables are at the same level or symmetric may be difficult.

Another method of passing two surgical cables sublamina of a vertebra includes securing a suture to a middle portion of the surgical cable, forming a loop in the surgical cable centered where the suture is attached, passing the suture sublamina of the vertebra, then pulling the suture through so that the surgical cable follows. Once passed, the surgical cable can be cut near where the suture was attached so that the final result is two surgical cables under the lamina of the vertebra. The cut is made to the surgical cable itself which may cause fraying of the new surgical cable ends. This latter shortcoming may cause difficulties in securing the surgical cable and may create other complications during the healing process. Because of the difficulty of using a frayed surgical cable with a fastener such as a crimp, and the increased chances for injury to surrounding tissue, frayed surgical cable ends are undesirable on either end of a surgical cable.

Therefore, a need has arisen for a leader that has good geometric control for passing two or more surgical cables through a selected region by guiding only one item or member through the region. A need has also arisen for a surgical cable leader and surgical cable end terminations that help prevent frayed ends.

### SUMMARY OF THE INVENTION

In accordance with the present invention, disadvantages and problems associated with previous systems and methods for installation of surgical cables have been substantially reduced or eliminated.

In accordance with one aspect of the present invention, a leader is provided for guiding surgical cables. The leader includes a stem having two or more arms extending from the stem and having surgical cables connected to each of the arms. In another aspect of the present invention, end terminations are provided for the surgical cable to prevent fraying of the surgical cable during installation and use.

A significant technical advantage of the present invention results from having a surgical cable assembly with a Y-leader which may be inserted through a restricted, sensitive portion of a patient's body such as the vertebrae foramen. The Y-leader provides improved control of the surgical cable assembly during installation and requires less space for the initial insertion.

### BRIEF DESCRIPTION OF THE DRAWINGS

For a more complete understanding of the present invention and the advantages thereof, reference is now made to the following description taken in conjunction with the accompanying drawings in which:
FIGURE 1 is a plan view of a surgical cable assembly with a surgical cable leader in accordance with the present invention;
FIGURE 2 is a schematic view of a surgical cable incorporating one embodiment of the present invention with a ball end termination and a monofilament end termination;
FIGURE 3 is a schematic view of a surgical cable incorporating another embodiment of the present invention with two ball end terminations;
FIGURE 4 is a schematic view of a surgical cable incorporating another embodiment of the present invention with two monofilament end terminations; and
FIGURE 5 is a schematic view of a surgical cable incorporating another embodiment of the present invention with two weld terminations.

### DETAILED DESCRIPTION OF THE INVENTION

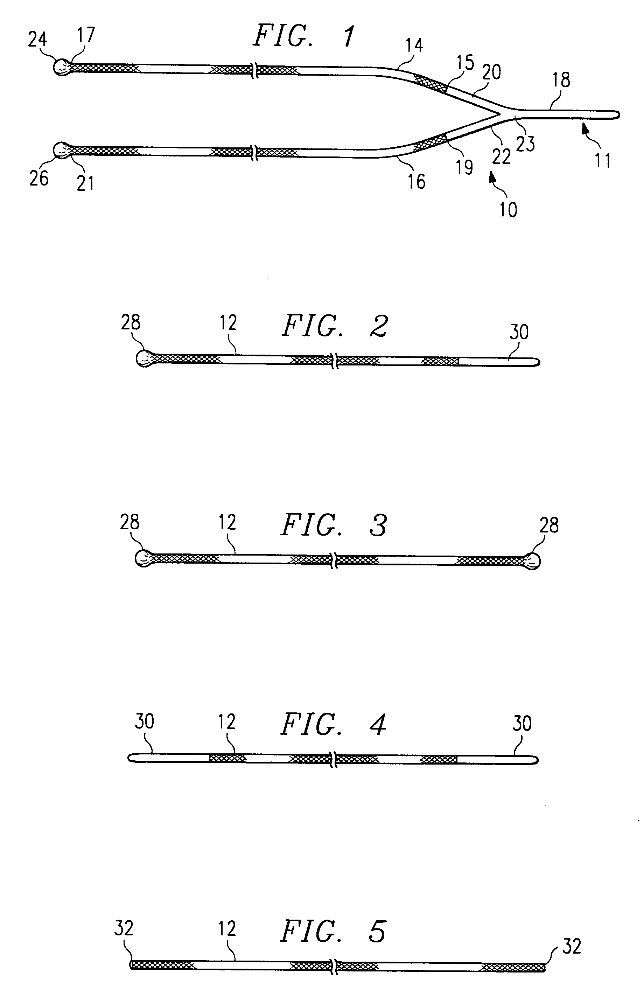

The preferred embodiments of the present invention and its advantages are best understood by referring to FIGURES 1 through 5 of the drawings, like numerals being used for like and corresponding parts of the various drawings.

Surgical cable assembly 10 incorporating one embodiment of the present invention is shown in FIGURE 1. Surgical cable assembly 10 includes first surgical cable portion 14 and second surgical cable portion 16. First surgical cable portion 14 has first end 15 and second end 17. Second surgical cable portion 16 has a first end 19 and a second end 21.

Surgical cable assembly 10 also includes leader 11. Leader 11 has stem 18 with a first arm 20 and a second arm 22 extending from one end. Arms 20 and 22 and stem 18 are preferably monofilament members. In other embodiments, stem 18 could have additional arms extending therefrom.

Stem 18 and arms 20 and 22 are arranged to form an angled configuration or a Y-configuration. Arms 20 and 22 may have a diameter smaller than the diameter of stem 18. For example, arms 20 and 22 may have half the diameter of stem 18 so that the diameter at fork 23 where arms 20 and 22 are connected to stem 18 is no greater in diameter than the other portions of surgical cable assembly 10.

First surgical cable portion 14 is secured at first end 15 to first arm 20 by a spot weld, and second surgical cable portion 16 is secured to arm 22 at first end 19 by a spot weld. While surgical cable portions 14 and 16 have been described as being attached to arms 20 and 22 by spot welding, those skilled in the art will understand that there are a multitude of other techniques that could be used, e.g., placing receptacle tubes on the ends of arms 20 and 22 that are crimped after surgical cable portions 14 and 16 have been placed inside.

Surgical cable assembly 10 has a first end termination 24 on second end 17 of surgical cable portion 14, opposite from end 15 of surgical cable portion 14 attached to arm 20. Likewise, surgical cable portion 16 has end termination 26 on second end 21, which is opposite from end 19 of surgical cable portion 16 attached to arm 22.

First and second end terminations 24 and 26 are shown as ball terminations. Other end terminations may be satisfactorily used with surgical cable assembly 10. For example, first and second end 17 and 21 may be ball terminations 28 as shown in FIGURE 3, monofilament terminations 30 as shown in FIGURE 4, or weld terminations 32 as shown in FIGURE 5. Of course, first end 17 and second end 21 could have any combination of the three terminations 28, 30, or 32; for example, a ball end termination 28 and a monofilament end termination 30 as shown in FIGURE 2.

Surgical cable portions 14 and 16 and leader 11 may be formed of titanium (6A14V) of Association of Testing Materials (ASTM) Specification F136, MP35N (ASTM Specification F162), or stainless steel. The preferred material for leader 11 is malleable, which allows stem 18 to be bent to take shapes that may further facilitate passing leader 11 through a selected region of a patient's body. Alternatively leader 11 may be formed of MP35N of Association of Testing Materials Specification F562, stainless steel, or ultra high molecular polyethylene. Cable portions 14 and 16 may also be formed of MP35N or stainless steel.

In a procedure using leader 11, stem 18 is guided through the selected region of the patient's body, e.g., sublamina of a vertebra. Surgical cable assembly 10 can be guided with control by passing only one member, leader 11, with approximately a one surgical cable thickness through the selected region. As stem 18 is guided, surgical cable portions 14 and 16 are guided since portions 14 and 16 are connected to stem 18 by arms 20 and 22. After passing stem 18 through the selected region, leader 11 may be cut at each arm 20 and 22 to form two separate surgical cables 14 and 16. Because arms 20 and 22 are preferably monofilament members, the cut ends will have monofilament end terminations and should not fray. Ends 17 and 21 opposite from arms 20 and 22 respectively will have any of three end terminations 28, 30 or 32. Having guided two or more surgical cables through the selected region, the surgeon can then continue with other procedures.

End terminations 28, 30 and 32 may be used with a surgical cable 12 that does not incorporate leader 11. For example, if only one surgical cable is to be passed through a selected region, a physician may desire to use surgical cable 12 with a monofilament end termination 30 and a ball end termination 28 as shown in FIGURE 2. Other combinations may be used as well, e.g., a ball termination 28 and a weld termination 32, a weld termination 32 and a monofilament termination 30.

Although the present invention and its advantages have been described in detail, it should be understood that various changes, substitutions and alterations can be made without departing from the spirit and the scope of the invention as defined by the following claims.

## Claims

1. A leader for guiding surgical cables in medical procedures comprising:
a stem having a plurality of arms extending from the stem; and
means for attaching one of the surgical cables to each arm extending from the stem.

2. The leader of Claim 1, wherein the stem comprises a monofilament member.

3. The leader of Claim 1, wherein the means for attaching comprises spot welding.

4. The leader of Claim 1, wherein the stem and plurality of arms comprise titanium.

5. The leader of Claim 1, wherein the stem and plurality of arms comprise MP35N.

6. The leader of Claim 1, wherein the stem and plurality of arms comprise ultra high molecular polyethylene.

7. The leader of Claim 1, wherein the stem and plurality of arms comprise stainless steel.

8. The leader of Claim 1, wherein the plurality of arms comprises a pair of arms.

9. Surgical cable assembly for use in medical procedures comprising:
a stem having a pair of arms extending from one end of the stem;
a portion of a surgical cable attached to each arm; and
the stem and arms further comprising a Y-configuration with the portions of the surgical cable extending from the Y-configuration.

10. The surgical cable assembly of Claim 9, wherein the elongated stem comprises a monofilament member.

11. The surgical cable assembly of Claim 9, wherein the stem and arms comprise stainless steel.

12. The surgical cable assembly of Claim 9, wherein the stem and arms comprise MP35N.

13. The surgical cable assembly of Claim 9, wherein the stem and arms comprise ultra high molecular polyethylene.

14. The surgical cable assembly of Claim 9, wherein the stem and arms are formed of titanium.

15. The surgical cable assembly of Claim 9, further comprising an end termination on the end of each surgical cable portion opposite the arm to which the surgical cable portion is attached.

16. The surgical cable assembly of Claim 15, wherein the end termination comprises a ball termination.

17. The surgical cable assembly of Claim 15, wherein the end termination comprises a weld termination.

18. The surgical cable assembly of Claim 15, wherein the end termination comprises a monofilament termination.

19. Surgical cable assembly for use in medical procedures comprising:
a stem having a first and second arm extending from one end of the stem;
the surgical cable assembly having a first and a second portion of surgical cable, the first portion of a surgical cable attached to the first arm, the second portion of a surgical cable attached to the second arm;
the stem and arms further comprising an angled configuration with the portions of the surgical cable extending from the angled configuration; and
end terminations formed on each surgical cable portion opposite the end of the surgical cable portion attached to the respective arms.

20. The surgical cable assembly of Claim 19, wherein the end terminations comprise ball terminations.

21. The surgical cable assembly of Claim 19, wherein the end terminations comprise monofilament terminations.

22. The surgical cable assembly of Claim 19, wherein the end terminations comprise weld terminations.
